Europäisches Patentamt

European Patent Office

Office européen des brevets

(11) Veröffentlichungsnummer: **0 446 463 A1**

# (12) EUROPÄISCHE PATENTANMELDUNG

(21) Anmeldenummer: 90124505.0

(22) Anmeldetag: 18.12.90

(51) Int. Cl.5: **A61J 1/00**, B65D 81/32, B67B 7/00

(30) Priorität: 15.03.90 DE 9002924 U

(43) Veröffentlichungstag der Anmeldung:
18.09.91 Patentblatt 91/38

(84) Benannte Vertragsstaaten:
AT BE CH DE DK ES FR GB GR IT LI LU NL SE

(71) Anmelder: BAYER AG

W-5090 Leverkusen 1 Bayerwerk(DE)

(72) Erfinder: Lietz, Hans
Bülsberger Höhe 2
W-5068 Odenthal(DE)
Erfinder: Buchmüller, Wilhelm
Ropenstall 58
W-5090 Leverkusen 3(DE)
Erfinder: Becker, Bernd, Dipl.-Ing.
Mühlenteichweg 13
B-4721 Kelmis(BE)

(54) Hohldorn aus Kunststoff.

(57) Der Hohldorn weist achsenparallele Kanäle (5, 6) zur Überführung des Inhaltes einer ersten Flasche in eine zweite Flasche auf und wird üblicherweise durch einen an der Flasche angeordneten, elastomeren Verschluß (2) hindurchgestochen. An seiner Peripherie ist der Hohldorn mit Verriegelungselementen (7) ausgestattet, die während des Durchstechens des Hohldorns durch den elastomeren Verschluß (2) nur eine in der Durchstichrichtung definierte Eindringtiefe des Dornes (1) erlauben und mit dem Verschluß (2) eine kraftschlüssige Verbindung eingehen.

FIG.1

EP 0 446 463 A1

Zur Übertragung eines Lösemittels aus einem Behältnis aus Glas oder Kunststoff bzw. auch aus einem Kunststoffbeutel in ein zweites Behältnis, in dem sich ein pharmazeutisches Präparat in trockener Form befindet, zur Herstellung einer pharmazeutischen Lösung sowie zur Entnahme dieser oder einer anderen Lösung zur Infusion werden Hohldorne benutzt, die in der Praxis auch als Überleitungskanülen oder Infusionsdorne bekannt sind. Zur Ausübung der beschriebenen Tätigkeiten werden die Dorne durch die die Behältnisse verschließenden elastomeren Dichtelemente hindurchgestochen.

Diese Dichtelemente, meist Stopfen oder Scheiben aus elastomeren Materialien, schmiegen sich fest an den Dorn an und sorgen so dafür, daß keine Flüssigkeit am Dorn entlang nach der Infusion ins Innere des Behältnisses gelangen können. Das Dichtelement dient ferner dazu, den Dorn für die Dauer der Infusion festzuhalten und gegen unbeabsichtigtes Herausziehen zu sichern. Solche Infusionsdorne gibt es in unterschiedlichen Ausführungen mit runden oder abgeflachten Spitzen, Spitzen mit Metalleinsätzen oder anderes. Das Material, aus dem solche Dorne hergestellt werden, kann Kunststoff oder Metall sein. Solche Dorne werden auch benutzt, um Flüssigkeiten in eine Flasche zu bringen, um den pulverförmigen oder gefriergetrockneten Inhalt zu lösen, um ihn anschließend infundieren zu können.

Beim Durchstechen der Dichtelemente erzeugen die bekannten Dorne Fragmente durch Abrieb, die in die Lösung gelangen und mit der Lösung als unerwünschte Verunreinigung in den Körper infundiert werden. Die Fragmentationsrate wird dabei deutlich beeinflußt von der Ausführung der Spitze des Dorns und von seiner Oberflächenbeschaffenheit. Die Spitzenausführung sowie die Oberflächenbeschaffenheit wirken sich auch auf die Kraft aus, die benötigt wird, um das Dichtelement zu durchstoßen. Die Oberfläche wirkt sich naturgemäß auch auf die Kraft aus, die benötigt wird, um den Dorn aus dem Dichtelement zu ziehen. Je glatter sie ist, desto leichter läßt sich der Dorn aus der Flasche ziehen, was für den Patienten naturgemäß eine große Gefahr bedeutet. Erschwerend in der Praxis kommt dazu, daß die Dorne oberflächlich mit einer leichten Schicht aus Silikonöl belegt sind, um sie leicht und möglichst ohne Reibung durch das Dichtelement stoßen zu können und damit die Fragmentationsrate klein zu halten. Durch diese Silikonölbelegung wird allerdings auch die Gefahr des unbeabsichtigten Herausziehens des Dornes deutlich größer.

Ein weiterer Nachteil der bekannten Hohldorne liegt darin, daß beim Durchdrücken des Dornes durch das elastische Dichtelement eine der Durchdrückrichtung entgegengesetzte Kraft auftritt, die den Dorn nach dem Nachlassen der Durchdringungskraft teilweise zurückdrückt, wodurch Kanäle, deren Öffnungen von der Spitze weiter entfernt sind, durch das Zurückgleiten des Dornes in das Dichtelement ganz oder teilweise verschlossen werden können.

Ziel der vorliegenden Erfindung war es also, einen Infusionsdorn zu entwickeln, der die aufgezählten Nachteile nicht aufweist.

Dieses Ziel wird erfindungsgemäß dadurch erreicht, daß der Hohldorn an seinem äußeren Durchmesser ein oder mehrere Verriegelungselemente trägt, die während des Durchstechens des Hohldorns durch den elastomeren Verschluß nur eine in der Durchstechrichtung definierte Eindringtiefe des Dornes erlauben und mit dem Verschluß eine kraftschlüssige Verbindung eingehen.

Durch diese Maßnahme wird sowohl das durch das elastische Verhalten des Verschlusses bewirkte, teilweise Zurückgleiten des Dornes gegen die Einstichrichtung als auch ein unbeabsichtigtes Herausreißen des Dornes aus dem Dichtelement zuverlässig vermieden.

Vorteilhaft besteht das Verriegelungselement aus einer radialen Einschnürung am Hohldorn, in die der der Durchstichstelle benachbarte Bereich des elastomeren Verschlusses aufgrund seiner elastischen Eigenschaften einschnappt. Die Anbringung einer peripheren, radialen Einschnürung am Dorn führt dazu, daß der Dorn nur zu einem bestimmten, genau definierten Teil seiner Länge durch das Dichtelement gleiten kann, bis das Dichtelement bzw. der elastomere Verschluß in die Einschnürung einschnappt. Dieses Einschnappen bewirkt auch, daß der Dorn nicht mehr gegen die Einstichrichtung zurückgedrückt wird. Die Austrittsöffnungen der internen Kanäle sind in dieser Position frei und offen zugänglich für einen optimalen Durchfluß.

Wie schon beschrieben, resultiert aus dem Einschnappen des Dichtelementes in die Einschnürung des Dornes eine kraftschlüssige Verbindung zwischen diesen beiden Elementen, so daß der Dorn gegen ein unbeabsichtigtes Herausziehen, z.B. durch den Patienten während einer Infusion, gesichert ist.

Ferner ist es günstig, wenn die radiale, zylindrische Einschnürung an ihren beiden Enden, d.h. an den Übergangsstellen zur Mantelfläche des Dorns, kegelstumpfartig abgeschrägt ist, wobei das Verhältnis vom Dornaußendurchmesser zum Durchmesser der Einschnürung vorteilhaft etwa 1,2 zu 1 bis 1,3 zu 1 beträgt.

Es hat sich herausgestellt, daß durch diese spezielle Formgebung der Einschnürung die Abdichtung zwischen dem Dorn und dem Inhalt der Flasche verbessert wird, da der elastomere Verschluß (Gummistopfen) im Bereich der Einschnürung leicht komprimiert wird, wodurch der Anpreßdruck auf den Dorn - und damit die Dichtwirkung - erhöht wird.

Ein weiterer Vorteil dieser Formgebung besteht darin, daß aufgrund des verringerten Innendurchmessers im Bereich der Einschnürung, verglichen mit herkömmlichen Hohldornen, nicht mehr soviel Gummi verdrängt wird, so daß sich die Öffnung im Gummistopfen wieder weitgehend von selbst schließt, wenn der Dorn nach einer Infusionsbehandlung herausgezogen wird. Dies wirkt dem Ausfließen von Infusionslösung in Fällen unsachgemäßer Handhabung entgegen und trägt somit dazu bei, das Kontaminationsrisiko von Operationsräumen oder Krankenzimmern zu vermindern.

Eine alternative Ausführungsform ist dadurch gekennzeichnet, daß die Verriegelungselemente aus zwei oder mehreren am äußeren Durchmesser des Dornes radial angebrachten Nocken bestehen, die in korrespondierende, entgegengesetzte Vertiefungen an einer den Verschluß festhaltenden Überkappe einrasten und mit denen sie durch eine Drehung kraftschlüssig, sicher und fest verbunden werden. Auf diese Weise kann der Hohldorn bajonettartig mit der Kappe verbunden werden, so daß - ähnlich wie bei der zuvor beschriebenen Lösung - ein unbeabsichtigtes Herausziehen des Dornes mit hoher Sicherheit verhindert wird. In analoger Weise wird verhindert, daß der Dorn zurückwandert. Allerdings kann nicht, wie bei der zuerst beschriebenen Alternative, die Abdichtung verbessert und die Wiederabdichtung nach dem Herausziehen des Dornes beschleunigt werden.

Wie zuvor beschrieben, entstehen beim Durchstechen eines Dornes durch das Dichtelement Dichtungsmaterialfragmente durch "Ausstanzen", "Abhobeln" oder Reibung. Es liegt auf der Hand, daß die Fragmentationshäufigkeit umso stärker ist, je dicker die Durchstichstelle des Dichtelementes ist. Andererseits benötigt man bei einem einfachen zylindrischen Dorn eine gewisse Durchstichdicke, um eine Mindesthaltekraft des Dornes im Dichtelement zu erreichen.

Im Zuge der Erfindung kann durch die Fixierung des Dorns aufgrund der Verriegelung im Dichtelement bzw. in der das Dichtelement haltenden Überkappe die Stärke bzw. Dicke des Dichtelementes erheblich reduziert und damit auch die Fragmentationsrate herabgesetzt werden.

Das Fragmentationsrisiko kann weiterhin vermindert werden, wenn dafür Sorge getragen wird, daß die im wesentlichen kegelförmige Durchstichfläche des Hohldorns an den Einmündungen der Hohlkanäle verrundet ist.

Überraschend wurde gefunden, daß das Eindringen des Dorns in das Dichtelement durch Aufrauhung der Oberfläche erleichtert wird. Diese Maßnahme trägt also ebenfalls dazu bei, die zum Durchstechen des Gummistopfens benötigte Kraft zu verringern und im Zuge einer verminderten Friktion die Fragmentierung durch Gummiabrieb zu reduzieren Damit wird die früher vorgenommene Oberflächensilikonisierung des Dornes entbehrlich.

Die erfindungsgemäßen Hohldorne werden zweckmäßig aus Kunststoffen durch Spritzgießen hergestellt. Zu diesem Zweck können vorteilhaft folgende Materialien verwendet werden: Polystyrol und Copolymere, Polycarbonat, Polymethylmethacrylat, Polyester, Polyamid und Polyolefine.

Im folgenden werden Ausführungsbeispiele der Erfindung anhand von Zeichnungen näher erläutert. Es zeigen:

Fig. 1    einen Hohldorn, bei dem die Verriegelung mittels einer Einschnürung erfolgt, und

Fig. 2    einen Hohldorn, bei dem die Verriegelungselemente aus Nocken bestehen.

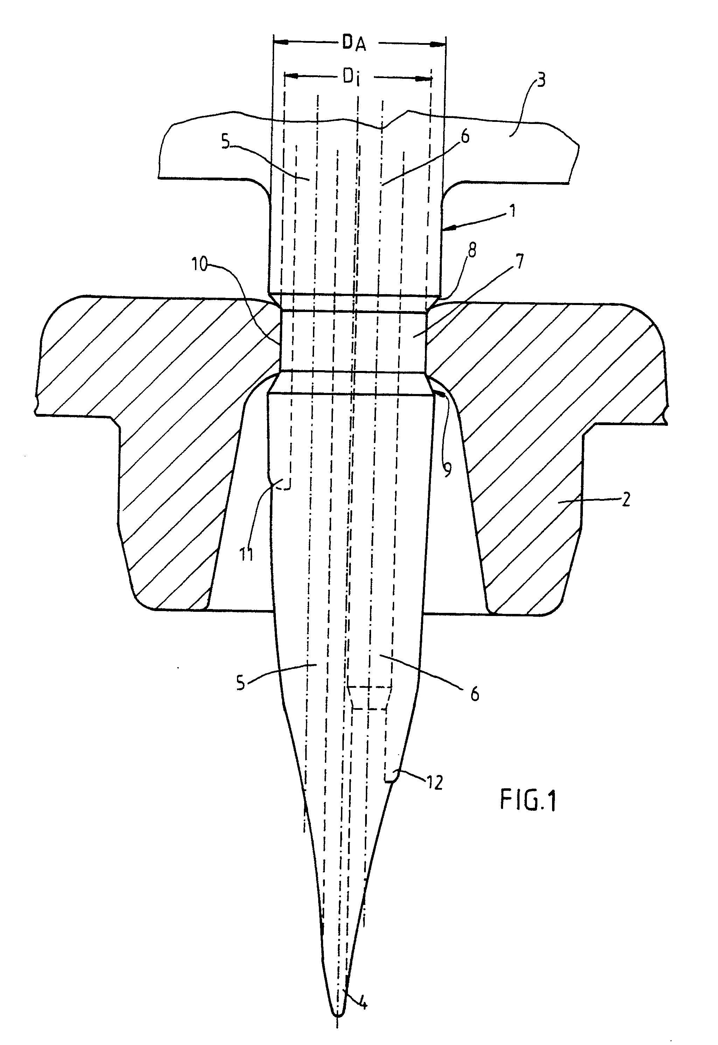

Gemäß Fig. 1 ist der Hohldorn 1 durch ein elastomeres Dichtelement 2 hindurchgestochen. Der Hohldorn 1 weist an seinem oberen Ende einen Griff 3 und an seinem unteren Ende eine im wesentlichen kegelförmige Spitze 4 auf. Der Kegelwinkel beträgt dabei ca. 24°. Zur Entnahme des Inhalts einer Infusionsflasche sowie zur Belüftung sind im Hohldorn 1 achsenparallele Kanäle 5 und 6 vorgesehen, die in die Mantelfläche der kegelförmigen Spitze 4 einmünden.

Der Hohldorn 1 weist ferner eine radiale Einschnürung 7 auf. Dabei wird eine Dimensionierung von $D_A$ durch $D_I$ von 1,2 zu 1 bis 1,3 zu 1 zugrundegelegt, wobei $D_A$ der Durchmesser des Hohldorns und $D_I$ der Durchmesser der Einschnürung 7 ist. Im Übergangsbereich zwischen dem reduzierten Durchmesser und dem vollen Außendurchmesser $D_A$ ist der Hohldorn 1 kegelstumpfartig angeschrägt (Kegelstumpfflächen 8 und 9). Die Flankenneigung der oberen Kegelstumpffläche 8 beträgt 40 bis 50° und die der unteren Kegelstumpffläche 950° bis 60°. Beim Durchschieben des Hohldorns 1 durch die zentrale Öffnung im elastomeren Dichtungselement bzw. Gummistopfen 2 schnappt der Gummistopfen 2 mit seiner peripheren Dichtungslippe 10 in die radiale Aussparung 7 am Hohldorn ein und wird dadurch fixiert. Aufgrund der speziellen Formgebung (abgeschrägte Flanken 8, 9 sowie Durchmesserverhältnis $D_A/D_I$) wird eine besonders gute Dichtwirkung erzielt.

Im Hinblick auf eine Minimierung des Fragmentationsrisikos beim Durchstechen des Hohldorns durch den Gummistopfen 2 sind die Übergänge 11, 12 von der Dornaußenwand auf die Hohlkanäle 5 und 6 verrundet.

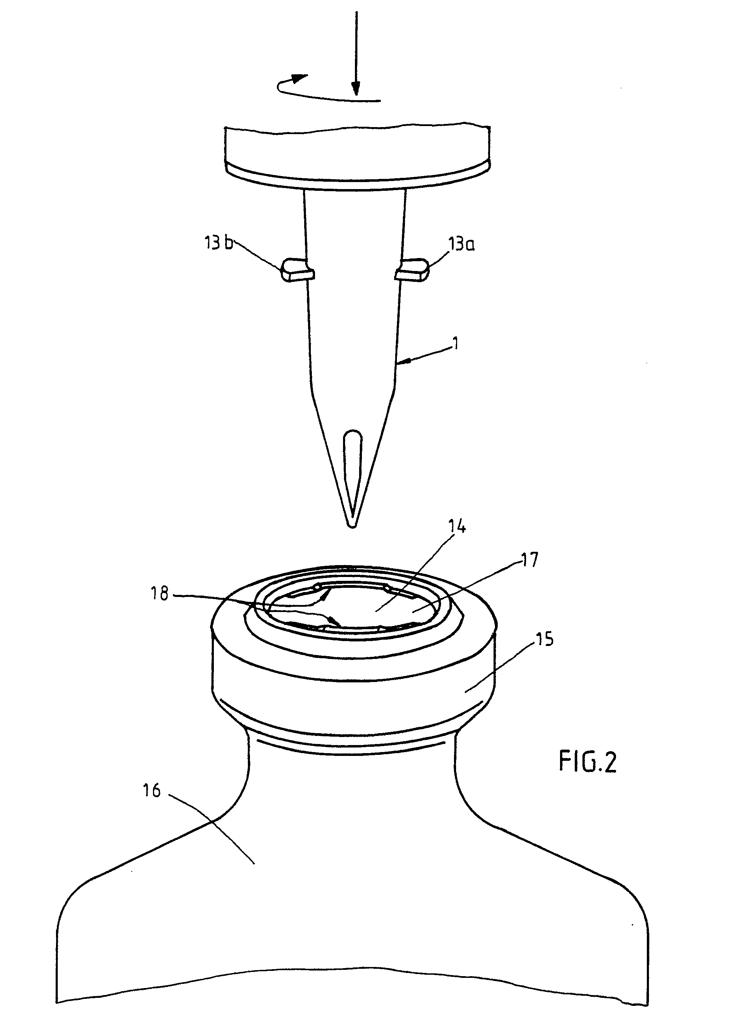

Die Fig. 2 zeigt die alternative Ausführung der Verriegelung des Hohldorns vor dem Durchstechen des Dichtelementes. In der Abbildung ist hier nur die Außenseite der als Dichtelement verwendeten Durchstechmembran zu erkennen. Der Dorn 1 ist hier mit radialen Nocken 13a, 13b versehen. Die Durchstichmembran 14 wird mit einer Überkappe 15 auf der Infusionsflasche 16 festgehalten. Die Überkappe weist eine zentrale Öffnung 17 auf, an deren Umfang Vorsprünge 18 angeordnet sind, die mit den Nocken 13a, 13b am Dorn 1 korrespondieren. Beim Durchstechen des Hohldorns 1 durch die Dichtungsmembran 14 rasten die Nocken 13 des Dorns in die Vorsprünge 18 ein, wenn der Dorn 1 gleichzeitig um seine Längsachse gedreht wird.

Auf diese Weise wird der Dorn 1 verriegelt und - analog zu der Ausführung nach Fig. 1 - am Flascheneintritt mechanisch fixiert.

In beiden Fällen ist die Oberfläche des Hohldorns 1 aufgerauht, da überraschenderweise nachgewiesen wurde, daß das Eindringen des Dorns in das Dichtelement 2 bzw. 14 durch die definierte Oberflächenrauhigkeit erleichtert wird. Die Hohldorne werden zweckmäßig aus Kunststoffen durch Spritzgießen hergestellt. Die Oberflächenrauhigkeit wird dabei durch eine negative Rauhigkeit der Formnester erzeugt, wobei die gewünschte Rauhigkeit mit einer Rauhtiefe von 4,5 $\mu$m gemäß VDI 3400/30 der Formnester durch funkenerosive Bearbeitung oder Strahlen mit Sand, Glasperlen oder dergleichen erreicht wird.

Anstelle zweier Kanäle 5, 6 kann der Hohldorn 1 auch mehrere, voneinander unabhängige, innere Kanäle aufweisen. Der Dorn 1 kann auch an seiner der Spitze 4 abgewandten Seite mit einem weiteren gleichachsigen Dorn fest verbunden sein, wobei beide Dorne entsprechend einer zur Mittelachse senkrecht stehenden Ebene symmetrisch angeordnet sind. Die beiden Dorne sind dabei gegenüber der senkrecht zur Mittelachse stehenden Ebene asymmetrisch angeordnet. Ferner kann der Hohldorn 1 anstelle eines kreisrunden einen ovalen bzw. elliptischen Querschnitt haben.

Beim Einsatz des erfindungsgemäßen Hohldorns wird dieser an seiner der Spitze 4 abgewandten Seite fest mit einer Tropfkammer verbunden, an der ein Infusionsbesteck angebracht ist.

**Patentansprüche**

1. Hohldorn aus Kunststoff, der zur Entnahme des Inhalts einer Medikamentenflasche (16) bzw. zur Überführung des Inhalts einer ersten Flasche in eine zweite Flasche achsenparallele Kanäle (5, 6) aufweist und durch einen an der Flaschenöffnung (17) angeordneten und die Flasche (16) dicht verschließenden, elastomeren Verschluß (2, 14) hindurchgestochen wird, dadurch gekennzeichnet, daß der Hohldorn (1) an seiner Peripherie ein oder mehrere Verriegelungselemente (7, 13a, 13b) trägt, die während des Durchstechens des Hohldorns durch den elastomeren Verschluß (2 bzw. 14) nur eine in der Durchstichrichtung definierte Eindringtiefe des Dornes (1) erlauben und mit dem Verschluß (2, 14) eine kraftschlüssige Verbindung eingehen.

2. Hohldorn nach Anspruch 1, dadurch gekennzeichnet, daß das Verriegelungselement aus einer radialen Einschnürung (7) am Hohldorn (1) besteht, in die der der Durchstichstelle benachbarte Bereich (10) des elastomeren Verschlusses (2) aufgrund seiner elastischen Eigenschaften einschnappt.

3. Hohldorn nach Anspruch 2, dadurch gekennzeichnet, daß die radiale, zylindrische Einschnürung (7) an ihren beiden Enden kegelstumpfartig abgeschrägt (8, 9) ist.

4. Hohldorn nach den Ansprüchen 1 bis 3, dadurch gekennzeichnet, daß das Verhältnis von Dornaußendurchmesser $D_A$ zum Durchmesser $D_I$ der Einschnürung (7) 1,2 zu 1 bis 1,3 zu 1 beträgt.

5. Hohldorn nach Anspruch 1, dadurch gekennzeichnet, daß die Verriegelungselemente aus zwei oder mehreren am äußeren Durchmesser des Dorns (1) radial angebrachten Nocken (13a, 13b) bestehen, die in korrespondierende, entgegengesetzte Vertiefungen 18 an einer den Verschluß (14) festhaltenden Überkappe (15) einrasten und mit denen sie durch eine Drehung kraftschlüssig fest verbunden werden.

6. Hohldorn nach den Ansprüchen 1 bis 5, dadurch gekennzeichnet, daß die im wesentlichen kegelförmige Durchstichfläche des Hohldorns (1) an den Einmündungen (11, 12) der Hohlkanäle (5, 6) verrundet ist.

7. Hohldorn nach den Ansprüchen 1 bis 6, dadurch gekennzeichnet, daß die Hohldornoberfläche aufgerauht ist.

8. Hohldorn nach den Ansprüchen 1 bis 7, dadurch gekennzeichnet, daß das Material des Hohldorns aus Polystyrol bzw. Copolymeren von Polystyrol oder Polycarbonat, Polymethylmethacrylat, Polyester, Polyamid oder Polyolefinen besteht.

FIG.1

13 b

13a

1

14

17

18

15

16

FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-U-8 913 766 (FRESENIUS) <br> * Seite 2, Zeile 14 - Seite 2, Zeile 23; Seite 3, Zeile 1 - Seite 3, Zeile 29; Seite 4, Zeile 1 - Seite 4, Zeile 6; Seite 6, Zeile 1 - Seite 6, Zeile 10; Seite 7, Zeile 19 - Seite 7, Zeile 30; Abbildungen 2,4 * <br> --- | 1-3,7 | A 61 J 1/00 <br> B 65 D 81/32 <br> B 67 B 7/00 |
| X | EP-A-0 021 405 (INTERMEDICAT) <br> * Seite 1, Zeile 31 - Seite 2, Zeile 15; Abbildungen 1-5 * <br> --- | 1,7,8 | |
| X | LU-A- 42 216 (HEDWIN CORP.) <br> * Seite 9, Zeile 1 - Seite 9, Zeile 21; Abbildungen 1-3,7,9,12 * <br> --- | 1,5 | |
| X | FR-A-2 300 578 (ILLINOIS TOOLWORKS) <br> * Seite 7, Zeile 30 - Seite 9, Zeile 9; Abbildungen 6-8 * <br> --- | 1 | |
| X | US-A-3 852 385 (HUGGINS) <br> * Spalte 5, Zeile 1 - Spalte 5, Zeile 5; Abbildung 2 * <br> --- | 1 | RECHERCHIERTE SACHGEBIETE (Int. Cl.5) |
| A | US-A-4 296 786 (THE WEST COMP.) <br> * Zusammenfassung; Abbildung 5 * <br> ----- | 1 | A 61 J <br> B 65 D <br> B 67 B <br> A 61 M |

Der vorliegende Recherchenbericht wurde für alle Patentansprüche erstellt

| Recherchenort | Abschlußdatum der Recherche | Prüfer |
|---|---|---|
| DEN HAAG | 16-05-1991 | BAERT F.G. |

EPO FORM 1503 03.82 (P0403)